# EUROPEAN PATENT APPLICATION

(11) **EP 3 366 346 A1**
(43) Date of publication of application: **29.08.2018**
(21) Application number: 16856970.5
(22) Date of filing: 14.10.2016
(51) Int. Cl.: A61N 1/32, C12N 5/0775

(54) **COSMETIC METHOD FOR REDUCING OR PREVENTING THE BUILD-UP OF FATTY TISSUE**

(30) Priority: 23.10.2015 ES 201531529
(71) Applicant: Indiba, S.A., 08192 Sant Quirze del Valles (Barcelona) (ES)
(72) Inventor: UBEDA MAESO, Alejandro, 28029 Madrid (ES); HERNANDEZ BULE, Maria Luisa, 28033 Madrid (ES)
(74) Representative: Durán-Corretjer, S.L.P.
(86) International application number: PCT/ES2016/070725
(87) International publication number: WO 2017/068214

(57) **Abstract**

The invention relates to a method for reducing or preventing the build-up of fatty tissue in an area of the human body, having a solely and exclusively cosmetic effect, comprising the application of alternating electric current with a frequency in the range of 0,4 and 0,6 MHz under subthermal conditions in said area of the human body.

## Description

The present invention relates to the field of cosmetics, more specifically it relates to a method for reducing or preventing the build-up of adipose tissue, having a solely and exclusively cosmetic, not therapeutic, effect.

Nowadays cosmetics makes use of treatments which use physical signals over a wide range of frequencies. In this regard, a wide variety of non-invasive techniques have been developed ranging from ultrasound to lasers, and which include electric and/or magnetic stimulation, either in the form of a direct current and low or ultra-low frequencies, or in the form of high frequencies or radiofrequencies (Mulholland, R.S., Paul M.D. and Chalfoun C., Clin Plast Surg, 2011, 38, 503-520, vii-iii; Belenky I., et al, Adv Ther, 2012, 29, 249-266). Specifically, treatments using radiofrequencies apply or use radiofrequency electromagnetic fields or electric currents in a frequency range approximately between 100 kHz and 3 GHz, which induce warming of the tissue by molecular friction.

In the field of cosmetics, the above-mentioned methods and treatments are applied, for example, to improve slackening or looseness of the skin, thus correcting and reducing wrinkles (Krueger N. and Sadick N.S., Cutis, 2013, 91, 39-46; Abraham M.T. and Mashkevich G., Facial Plast Surg Clin North Am., 2007, 15, 169-177, v.; Alster T.S. and Lupton J.R., Clin Dermatol, 2007, 25 487-491; Sadick N., Facial Plast Surg Clin North Am, 2007, 15, 161-167, v.; Chipps L.K. et al, J Drugs Dermatol, 12 (2013) 1215-1218; Tay Y.K. and Kwok C., J Cosmet Laser Ther, 11 (2009) 25-28). Another cosmetic application includes body shaping, of which the most visible or significant effect has been described as sculpting the figure by reducing cellulitis and subcutaneous adipose tissue (van der Lugt C. et al, Dermatol Ther, 22 (2009) 74-84; Del Pinto E. et al, J Drugs Dermatol, 5 (2006) 714-722, Alexiades-Armenakas M., Dover J.S. and Arndt K.A., J Cosmet Laser Ther, 10 (2008) 148-153; and Valentim da Silva R.M. et al, Dermatol Res Pract, 2013 (2013) 715-829).

This last effect (body sculpting and reducing fat deposits) has been demonstrated with satisfactory results in experimental studies on rabbits treated with electrothermal radiofrequency currents, a reduction in the number of dermal and hypodermal adipocytes being observed, together with an increase in the density of the connective tissue (Ronzio, O.A., Fisioterapia, vol. 31, 2009, pp. 131-136).

Clinical studies have also reported satisfactory results from radiofrequency treatments applied to the reduction of fat deposits and an improvement in post-partum slackness of the skin (Brightman L., et al, Lasers Surg Med, 41 (2009) 791-798). At tissue level, it has been shown that the non-invasive, percutaneous application of radiofrequency treatments produces a warming of the subcutaneous tissue, which results in the restructuring of the collagen fibres and an increase in microcirculation in the adipose tissue, both in animal subjects (Belenky I. et al, Adv Ther, 29 (2012) 249-266) and in human beings (Trelles M.A. et al, Lasers Med Sci, 25 (2009) 191-195). At cellular level, electrothermal radiofrequency treatment, depending on the frequency and power of the signal, may cause the lipolysis and necrosis of adipocytes (Trelles M.A. et al, Lasers Med Sci, 25 (2009) 191-195; Hamida Z.H. et al, Appl Physiol Nutr Metab, 36 (2011) 271-275).

Electrotherapy based on the electrothermal technology known as Capacitive and Resistive electric transfer (CRet) consists of a non-invasive strategy based on the use of alternating currents with frequencies in the range 0.4 MHz to 0.6 MHz (a range comprised in the radiofrequency spectrum) to raise the temperature of the target organs or tissues for treatment by the action of said alternating electric currents. This type of technology has been shown to be effective in medical rehabilitation and regeneration treatments, and also in aesthetic/cosmetic applications, for example, for the regeneration of lesions produced by injury/injuries or degenerative lesions of the tissues, by reducing the associated pain, reducing inflammation, increasing blood circulation, improving vascular and muscular tone and improving the reabsorption of haematomas, oedemas and liquid that has built up in the joints and soft tissues.

In the case of CRet therapy, the treatment is applied manually, by applying pressure with capacitive or resistive electrodes on the skin, so that the underlying target tissues receive three simultaneous stimuli: one thermal, another electrically induced, and a third mechanical. The prior art to date had shown that cosmetic treatment using CRet induces or produces a lipolytic or antiadipogenic effect, which until now had been attributed exclusively to the effect that the combination of the thermal and mechanical stimuli produced in the tissues to which the treatment is applied, the assumption therefore being that the alternating radiofrequency electric current had no effect on the tissue other than that of increasing the temperature thereof.

Patent application PCT/ES2015/070372 discloses the use or application of radiofrequency alternating electric current *in vitro,* which by itself and under sub-thermal (electric) conditions, increases the proliferation of mesenchymal stem cells derived from subcutaneous adipose tissue without affecting the ability thereof to be differentiated into various cell types, such as osteocytes, adipocytes or chondrocytes.

In addition, in recent years numerous studies and investigations have been conducted based on the use of the above-mentioned electrotherapy under sub-thermal conditions in order to analyse the effect of the electric component on the cells and rule out possible oncogenic or tumour development risks. Said studies have demonstrated that the electric component of the therapy effectively has an effect on the cells such that, when applied to cultures of tumour cell lines, cytostatic or cytotoxic effects are observed. Specifically, it has been shown that:
- An alternating electric current with a frequency of 0.57 MHz applied under sub-thermal conditions (5-minute pulses at 0.57 MHz with a current density of 50 µA/mm², applied every 4 hours over a period of 12 to 24 hours) to cell cultures of the HepG2 cell line (ATCC deposit no. HB-8065) derived from human hepatocarcinoma cells, produces a cytostatic and cell differentiation effect thereon (Hernandez-Bule, M.L. et al, Int J Oncol, 2007, 30, 583-592; Hernandez-Bule, M.L. et al, Int J Oncol, 2010, 37, 1399-1405; and Hernandez-Bule, M.L. et al, PLoS ONE, 2014, 9, 1e84636).
- An alternating electric current with a frequency of 0.57 MHz applied under sub-thermal conditions (5-minute pulses at 0.57 MHz with a current density of 50 µA/mm², applied every 4 hours over a period of 12 to 24 hours) to cell cultures of the NB69 cell line (Sigma catalogue no. 99072802 at Sigma-Aldrich) derived from human neuroblastoma cells, produces a cytotoxic effect (Hernandez-Bule, M.L. et al, Int J Oncol, 2012, 41, 1251-1259).

As well as said anti-tumour effects, it has also been disclosed that an alternating electric current with a frequency of 0.57 MHz applied under sub-thermal conditions (5-minute pulses at 0.57 MHz with a current density of 50 µA/mm², applied every 4 hours over a period of 12 to 24 hours) does not produce any detectable effect in cell cultures of peripheral blood mononuclear cells from healthy donors, given that no statistically significant changes were detected in the survival, necrosis and distribution of cell subpopulations after treatment with said alternating electric current (Hernandez-Bule, M.L. et al, Int J Oncol, 2012, 41, 1251-1259).

The inventors of this patent, following extensive and exhaustive experiments, have discovered surprisingly that the application *in vitro* of alternating electric current with a frequency of in the range 0.4 to 0.6 MHz under sub-thermal conditions, allows the build-up of fat during the process of adipogenesis of adipose tissue stem cells to be reduced and prevented.

A first aspect of the present invention relates to a cosmetic method for reducing or preventing the build-up of adipose tissue in an area of an adult human body, which method comprises the application of alternating electric current with a frequency in the range 0.4 to 0.6 MHz under sub-thermal conditions. Said method of the present invention has solely and exclusively cosmetic, and not therapeutic, effects.

In an additional aspect, an alternating electric current generating device is also disclosed which is configured to carry out the cosmetic method of the present invention.

In another aspect, the present document also discloses the use of an alternating electric current generating device to carry out the cosmetic method of the present invention.

As used in the present document, 'adult' and the plural thereof is used to refer to persons aged at least 16 years.

As used in the present document 'visceral adipose tissue' and the plural thereof is used to refer to adipose tissue which is found on a mediastinal, mesenteric, perigonadal, perirenal and retroperitoneal scale.

Accordingly, as mentioned earlier, in a first aspect, the present invention relates to a method for reducing or preventing the build-up of adipose tissue in an area of a human body, having a solely and exclusively cosmetic effect, characterised in that said method comprises the application of alternating electric current with a frequency in the range 0.4 to 0.6 MHz under sub-thermal conditions in said area of the human body.

The cosmetic method of the present invention is a non-invasive, non-traumatic cosmetic method with percutaneous application of sub-thermal densities of an alternating electric current emitted by electrodes applied to the skin, in order to reduce adipose tissue subjected to the action of the current.

The cosmetic method of the present invention also helps both to reduce and prevent the build-up of adipose tissue, since the application of alternating electric current under the conditions indicated above has an antiadipogenic effect in the initial and intermediate stages of adipocyte differentiation.

The cosmetic method of the present invention may be carried out on a male or female, preferably adult, human body.

In a preferred embodiment, the alternating electric current has a frequency in the range 0.4 MHz to 0.5 MHz, more preferably with a frequency of 0.448 MHz.

As mentioned earlier, the conditions relating to treatment time and current density are established in such a way that the method is carried out under sub-thermal conditions (that is, only the electric effect of the current applied is produced without inducing a rise in temperature). Preferably, in the cosmetic method of the present invention the density of the alternating electric current is between 1 and 3000 µA/mm², more preferably between 5 and 500 µA/mm²; for between 6 and 20 application sessions of alternating electric current, more preferably between 8 and 15 application sessions of alternating electric current; in applications of between 1 and 5 sessions for the application sessions of alternating electric current per week, still more preferably in applications of 3 application sessions of alternating electric current per week; with each alternating electric current application session lasting between 30 and 90 minutes, more preferably 60 minutes.

In a preferred embodiment, the cosmetic method of the present invention is applied to the skin and is carried out using a pair of electrodes made of a suitable material (for example, stainless steel with or without an electrically insulating coating) situated on the area of the human body concerned and connected to an alternating electric current generating device. The connection of the electrodes to the alternating electric current generating device may be serial or parallel. Examples of alternating electric current generating devices which with a suitable configuration may be used to carry out the cosmetic method of the present invention are the following devices produced by Indiba®: the Activ 902, Activ HCR 902 and ELITE models, among others.

It is envisaged that the cosmetic method of the present invention may be applied to any area of the human body which has, or which is likely to have, a build-up of adipose tissue. In a preferred embodiment, the cosmetic method of the present invention is used to reduce or prevent the build-up of adipose tissue in the abdomen, gluteus, thighs, waist, back, arms, 'saddlebags' or combinations thereof, more preferably in the abdomen, 'saddlebags' or combinations thereof.

The area to be treated, as described earlier, is placed between electrodes made of a suitable material.

One of the main advantages of the cosmetic method of the present invention is the fact that, unlike conventional methods based on thermal or mechanical components which only serve to reduce the subcutaneous adipose tissue that comes in contact with the stimulus, the cosmetic method of the present invention affects all the adipose tissue (reducing or preventing its formation) situated between the electrodes used, both the subcutaneous adipose tissue and the visceral adipose tissue.

Moreover, the cosmetic method of the present invention is used to reduce white adipose tissue, brown adipose tissue or combinations thereof, preferably, white adipose tissue.

As mentioned previously, the present cosmetic method is used to reduce built-up adipose tissue, to prevent it building up, or combinations thereof. The use or the effect will depend on the state of the human body on which the cosmetic method of the present invention is used, that is, if there is already a pre-existing build-up of adipose tissue or if, on the contrary, the method is clearly being used to prevent future build-ups. However, in a preferred embodiment, the cosmetic method is used to prevent the build-up of adipose tissue in the area of the human body on which said method is used.

The cosmetic method of the present invention may be used together with other cosmetic methods or treatments which have antiadipogenic effects by reducing or preventing the build-up of adipose tissue, so that the effects thereof may act additionally or synergistically. In a preferred embodiment, the cosmetic method of the present invention is combined with thermal, electric or mechanical stimuli, or combinations thereof.

The present document discloses an alternating electric current generating device which comprises a control device configured to carry out the cosmetic method of the present invention.

Said device preferably comprises a pair of electrodes made of a suitable material (for example, stainless steel with or without an electrically insulating coating) connected to the alternating electric current generating device, preferably, serially or in parallel. More preferably, the electrodes are connected serially.

The control device comprised in the alternating electric current generating device is configured to generate alternating electric current at a frequency in the range 0.4 MHz to 0.6 MHz, preferably in the range 0.4 MHz to 0.5 MHz, still more preferably a frequency of 0.448 MHz.

As mentioned earlier, the treatment time and current density conditions in the cosmetic method of the present invention are established in such a way that the method is carried out under sub-thermal conditions (that is, only the electric effect of the current applied is produced without inducing a rise in temperature), therefore, the control device comprised in the alternating electric current generating device is configured to generate alternating electric current and apply said current under sub-thermal conditions. Preferably, the control device comprised in the alternating electric current generating device is configured so that the density of the alternating electric current generated by the alternating electric current generating device is between 1 and 3000 µA/mm², still more preferably between 5 and 500 µA/mm²; and for said alternating electric current to be applied in pulses or individual sessions lasting between 30 and 90 minutes, preferably 60 minutes.

The control device configured to carry out the cosmetic method of the present invention may comprise a memory which contains the programming required to carry out the method of the present invention as indicated above.

In the present document the use of an alternating electric current generating device to carry out the cosmetic method of the present invention is also disclosed.

Said alternating electric current generating device is configured to carry out the method of the present invention, as indicated above.

For a better understanding, the present invention is described in more detail below with reference to the accompanying drawings which are given by way of example, and with reference to non-limiting, illustrative examples. Said illustrative examples show *in vitro* results relating to genes, proteins and cellular pathways that are relevant to the process of adipogenesis in humans (that is, *in vivo*) and therefore validate the application of the cosmetic method of the present invention in humans.

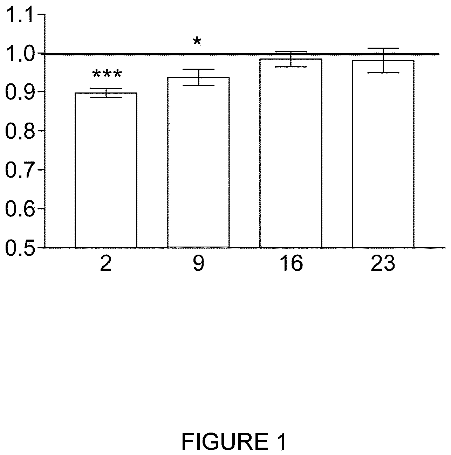

Fig. 1 shows the results obtained for the quantity of fatty acids present in each of the groups kept in an adipogenic differentiation medium for 2, 9, 16 or 23 days and treated with pulses of alternating electric current under sub-thermal conditions for the final 48 hours in the above-mentioned differentiation medium. Said quantity is determined by staining with Oil Red O and measuring absorbance at 510nm. The data in this figure are expressed as a decimal of the corresponding differentiated control group. The ordinate (y axis) shows absorbance at 510nm and the abscissa (x axis) shows the group, that is, the number of days for which the culture of stem cells derived from adipose tissue was in the adipogenic differentiation medium. In this and the following figures, the asterisks denote statistically significant levels, calculated using Student's t test, in the differences between the treated samples and the respective controls. *:0.01 ≤ p < 0.05; **: 0.001 ≤ p < 0.01; ***: p < 0.001.

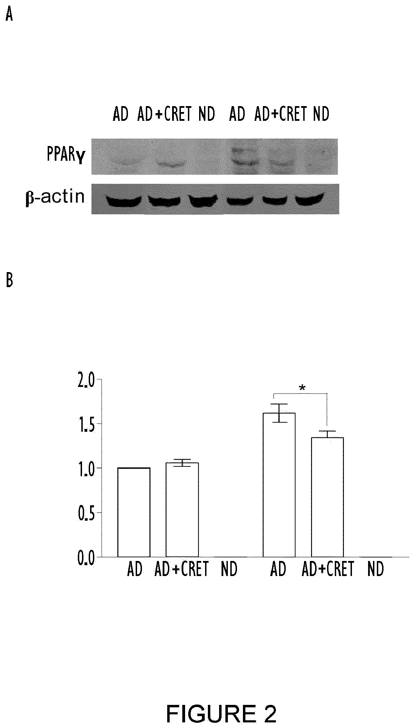

Fig. 2 shows the results obtained for the expression of PPAR-γ protein in the control (AD) and treated (AD + CRet) groups, and in stem cells derived from adipose tissue that were not subjected to the adipogenic differentiation treatment (ND). In Fig. 2A, an immunoblot is shown in which the first row corresponds to the PPAR-γ protein and the second row corresponds to the β-actin protein, used as a load control. Fig. 2B shows the densitometric analysis for PPAR-γ strips of the immunoblot shown in Fig. 2A. In said Fig. 2B, the results appear expressed as a decimal of the density of the PPAR-γ strip observed for the differentiated cell control group over 2 or 9 days. In Fig. 2B, the ordinate (y axis) shows the relative density (proportional to the expression of the protein in question) and the abscissa (x axis) shows the group to which the results refer. Both in Fig. 2A and 2B the first three columns refer to cultures that were differentiated over 2 days (or maintained in culture during this period without differentiation in the case of the ND group) and the last three columns refer to cultures that were differentiated over 9 days (or maintained in culture during this period without differentiation in the case of the ND group).

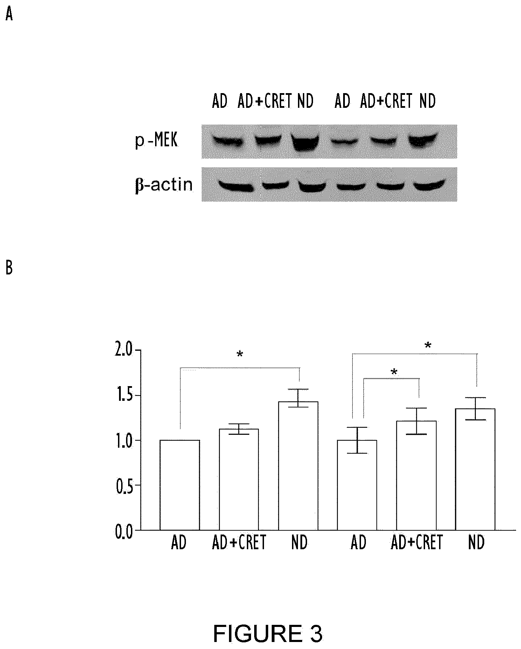

Fig. 3 shows the results obtained for the expression of the p-MEK protein in the control (AD) and treated (AD+CRet) groups and in stem cells derived from adipose tissue that were not subjected to the adipogenic differentiation treatment (ND). In Fig. 3A an immunoblot is shown in which the first row corresponds to the p-MEK protein and the second row corresponds to the β-actin protein, used as a load control. Fig. 3B shows the densitometric analysis for p-MEK strips of the immunoblot shown in Fig. 3A. In said Fig. 3B, the results appear expressed as a decimal of the density of the p-MEK strip observed for the control group of cultures of stem cells derived from adipose tissue that were differentiated over 2 or 9 days. In Fig. 3B, the ordinate (y axis) shows the relative density (proportional to the expression of the protein in question) and the abscissa (x axis) shows the group to which the results refer. Both in Fig. 3A and 3B the first three columns refer to cultures that were differentiated over 2 days (or maintained in culture during this period without differentiation in the case of the ND group) and the last three columns refer to cultures that were differentiated over 9 days (or maintained in culture during this period without differentiating in the case of the ND group).

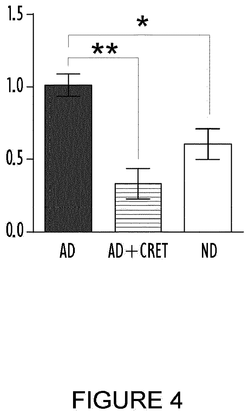

Fig. 4 shows the immunofluorescence results obtained for PPAR-γ in groups treated with alternating electric current under sub-thermal conditions and control groups of cultures of stem cells derived from adipose tissue differentiated for 9 days. The black column corresponds to the differentiated control group (AD), the column with horizontal lines corresponds to the treated group (AD+CRet) and the white column corresponds to stem cells derived from adipose tissue that were not subjected to adipogenic differentiation treatment (ND). The results appear expressed as a decimal of what was observed in the control group that was differentiated for 9 days. The ordinate (y axis) shows the normalisation of the quantity of nuclei which express PPAR-γ (PPAR-γ+) and the abscissa (x axis) shows the experimental group.

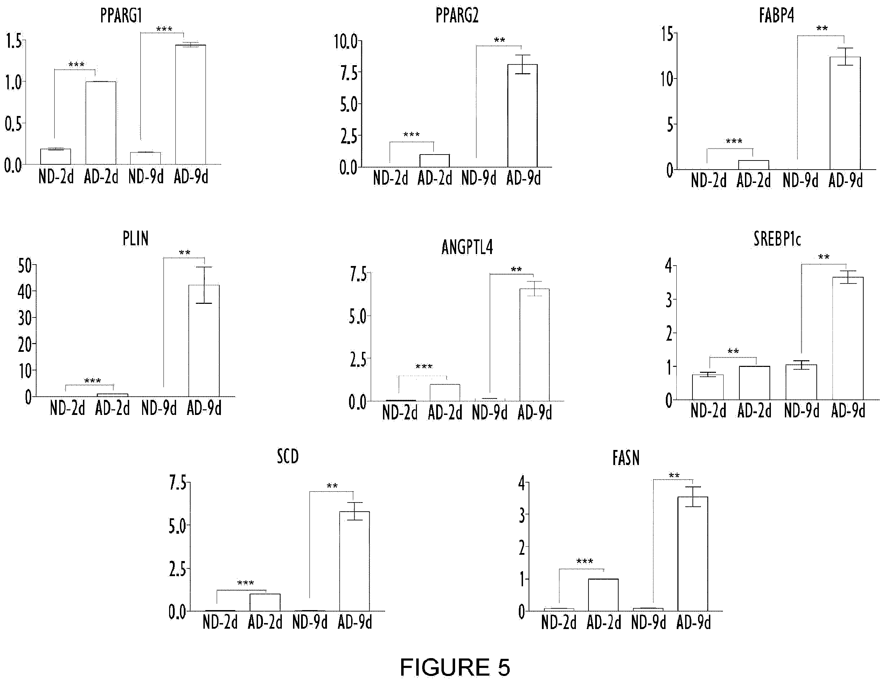

Fig. 5 shows the differences of expression in the PPARG1, PPARG2, FABP4, PLIN, ANGPTL4, SREBP1c, SCD and FASN genes observed in the adipogenic differentiation method in stem cells derived from adipose tissue. In all the graphs the same column structure is followed, from left to right: 2 days with no differentiation treatment; 2 days with differentiation treatment; 9 days with no differentiation treatment; and 9 days with differentiation treatment. The results are expressed as a decimal of what was observed for the culture of stem cells derived from adipose tissue with differentiation treatment lasting 2 days. In all the graphs the ordinate (y axis) shows the relative expression of the corresponding messenger RNA and the abscissa (x axis) shows the group.

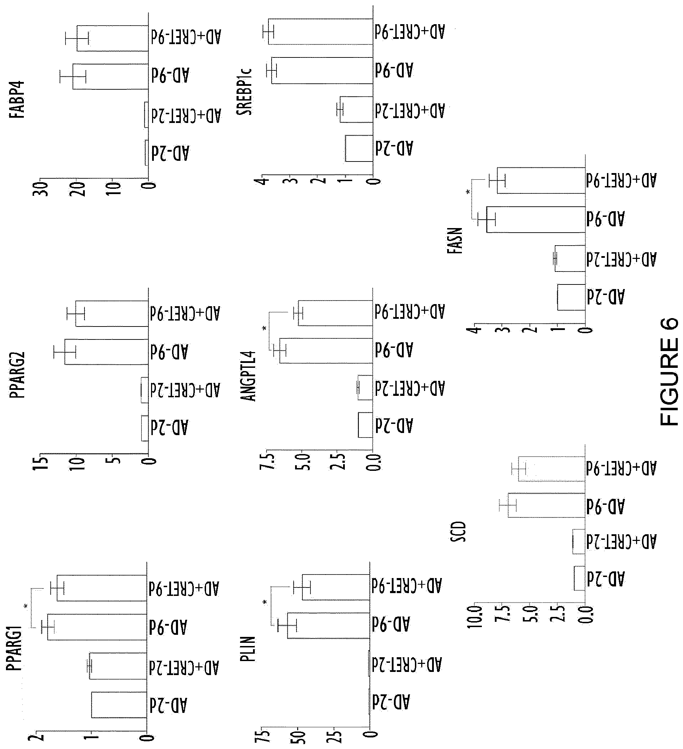

Fig. 6 shows the differences of expression in the PPARG1, PPARG2, FABP4, PLIN, ANGPTL4, SREBP1c, SCD and FASN genes observed between control groups (stem cells derived from adipose tissue subjected to adipogenic differentiation) and treated groups (stem cells derived from adipose tissue subjected to adipogenic differentiation and treated with alternating electric current under sub-thermal conditions). In all the graphs the same column structure is followed, from left to right: 2 days with no alternating electric current treatment; 2 days with alternating electric current treatment; 9 days with no alternating electric current treatment; and 9 days with alternating electric current treatment. The results are expressed as a decimal of what was observed for the culture of stem cells derived from adipose tissue subjected to adipogenic differentiation treatment for 2 days with no alternating electric current treatment. In all the graphs the ordinate (y axis) shows the relative expression of the corresponding messenger RNA and the abscissa (x axis) shows the group.

### Example 1. Obtaining and culture of stem cells derived from adipose tissue

The stem cells derived from adipose tissue were isolated from subcutaneous adipose tissue obtained surgically from healthy donors: men and women between 29 and 69 years of age. The isolation protocol has already been described in detail in the prior art (Hernandez-Bule, M.L., Cell Physiol Biochem, 2014, 34, 1741-1755). Briefly, the protocols for informed consent and for the collection and processing of the samples met the applicable ethical standards in the European Union and were evaluated and approved by the ethical committee for clinical tests of the Hospital Universitario Ramón y Cajal. The stem cells derived from adipose tissue were isolated from pieces of fat measuring 0.5-1 cm³, free from the remains of blood vessels and fibrotic tissue and cut into fragments measuring 1-2mm³. Said fragments were digested with collagenase A (Roche Applied Science, Basel, Switzerland) at a concentration of 1mg/ml for 40 minutes at 37°C. The digested tissue was dissociated using a P1000 pipette. The resulting cell dispersion was centrifuged at 300 x g for 5 minutes to isolate the stromal vascular fraction. The resulting sediment or pellet was re-suspended in a suitable culture medium (MesenPro-RSTM, Gibco, Invitrogen, Camarillo, CA, USA) supplemented with 1% of glutamine (Gibco, Invitrogen, Camarillo, CA, USA) and 1% of penicillin-streptomycin (Gibco, Invitrogen, Camarillo, CA, USA) and the cells present in said sediment were seeded in a 75 cm² T culture flask (Falcon, Corning, NY, USA). After 4 days culture, the medium was renewed, and 3 days later, when the cells were confluent, said cells were sub-cultured. Accordingly, the cells were detached using 0.05% trypsin with 0.02% EDTA (Sigma-Aldrich, St Louis, MO, USA) in Hank's saline solution and seeded in a new 75 cm² T culture flask at a density of 670 cells/cm².

All the following examples used cells obtained in accordance with this example, in passages 3 to 7, and which were cultured in 60 mm Petri dishes (Nunc, Roskilde, Denmark) at a density of 2270 cells/cm².

### Example 2. Effect of the treatment with alternating electric current under sub-thermal conditions on the lipid content during the adipogenic differentiation of stem cells derived from adipose tissue.

As mentioned earlier, the stem cells derived from adipose tissue obtained in accordance with example 1, in passages 3 to 7, were cultured in 60 mm Petri dishes (Nunc, Roskilde, Denmark) at a density of 2270 cells/cm².

After 4 days of growth in Petri dishes, the cultures were incubated in adipogenic differentiation medium made up of D-MEM with a high glucose content (Biowhittaker, PA, USA) supplemented with 10% of foetal bovine serum (Gibco, Invitrogen, Camarillo, CA, USA), 1% of glutamine and 1% of penicillin-streptomycin (Gibco, Invitrogen, Camarillo, CA, USA), 3-isobutyl-1-methylxanthine at a concentration of 0.25 mM (IBMX, Gibco, Invitrogen, Camarillo, CA, USA), indomethacin at a concentration of 200 µM (Sigma-Aldrich, St Louis, MO, USA), insulin at a concentration of 10 µg/ml (Sigma-Aldrich, St Louis, MO, USA) and dexamethasone at a concentration of 1 µM (Sigma-Aldrich, St Louis, MO, USA). The cultures were kept in this medium for the desired differentiation time: 2, 9, 16 or 23 days, replacing the medium with new medium every 3-4 days. The cultures were treated with alternating electric current (CRet) or simply incubated in the presence of electrodes connected to the device (controls to which alternating electric current was not applied) for the final 48 hours of adipogenic differentiation treatment.

Said CRet treatment and the system which were used have been described in detail in the prior art (Hernandez-Bule, M.L. et al, Int J Oncol, 2010, 37, 1399-1405; and Hernandez-Bule, M.L. et al, Int J Oncol, 2007, 30, 583-592). Briefly, the exposure or treatment with alternating electric current was carried out using pairs of sterile stainless steel electrodes designed for the purpose of *in vitro* stimulation. Said electrodes were housed in all the Petri dishes (which contained cultures of stem cells derived from adipose tissue), both those belonging to the groups treated with CRet (alternating electric current) and the control groups in which, as indicated earlier, the electrodes connected to the alternating electric current generating device were simply positioned but without applying the current. Only cultured cells in the rectangular area situated within the area delimited by the electrodes were used in the present study (cells situated on the remaining surface of the dish were disregarded).

For the exposure to alternating electric current, the pairs of electrodes were connected serially to an alternating electric current generator (Indiba Activ 902 model, INDIBA®, Barcelona, Spain).

As mentioned earlier, in the control groups, the pairs of electrodes were also inserted in the dishes. Said electrodes were also connected to the alternating electric current generating device, but said device was not actuated.

For the groups exposed to or treated with alternating electric current, the stimulation pattern consisted of 5-minute pulses of alternating electric current at a frequency of 0.448 MHz with a current density of 50 µA/mm², separated by 4-hour pauses or rests between pulses, for a total of 48 hours. This method ensures that the electric treatment is applied under sub-thermal conditions.

During the 48-hour treatment interval, each treated group and the corresponding control group were cultured simultaneously, separated in two identical CO2 incubators (Thermo Fisher Scientific, Waltham, MA, USA). The treatment parameters, and the atmospheric conditions inside the incubators (temperature of 37°C, relative humidity of 90% and partial CO² pressure of 5%) were monitored constantly. The electromagnetic environment inside the incubators was controlled by means of special magnetometers for three frequency ranges of interest: static, industrial frequency (50 Hz and the harmonics thereof) and radiofrequency. The values recorded coincided with those reported in the prior art and corresponded to field levels typically found in laboratory environments.

As mentioned earlier, various groups were formed depending on the differentiation time: 2, 9, 16 or 23 days of adipogenic differentiation. For each of said time frames, the set of Petri dishes was divided into three groups. Two of these groups were differentiated; one was treated with alternating electric current and the other served as a differentiated control. The remaining group continued without differentiation for the corresponding time intervals. The treatment with alternating electric current began 48 hours before the end of the incubation of the culture in adipogenic differentiation medium, that is, at 0, 7, 14 and 21 days respectively.

Once the culture and treatment of the above-mentioned groups was carried out, the quantities of fatty acids synthesised by the cultures incubated in adipogenic differentiation medium for 2, 9, 16 or 23 days as indicated above, were quantified in order to evaluate the cellular response to the adipogenic action of the differentiation medium and to the treatment with alternating electric current. Accordingly, after treatment (alternating electric current or control), the cultures were washed with phosphate-buffered saline and fixed in 4% paraformaldehyde at 4°C for 20 minutes. Next, the cells were permeabilised by treatment with 60% isopropanol for 3 minutes and then the cultures were stained with Oil Red O (Sigma-Aldrich, St Louis, MO, USA) for 30 minutes.

After staining, 15 microscope fields were randomly selected in each Petri dish and photographed.

The stained fatty acids were extracted by stirring the samples in 99% isopropanol for 5 minutes, and the fatty acid content was evaluated by spectrophotometry at 510 nm.

Fig. 1 shows the summarised results obtained for the quantification of the fatty acids. In the cultures of stem cells derived from subcutaneous adipose tissue which are in the early or intermediate adipogenic differentiation stages (cultures treated or incubated in adipogenic differentiation medium for 2 or 9 days) a statistically significant reduction in the build-up of fatty acids in the cultures is observed. In contrast, when the cultures are in the advanced stages of adipogenic differentiation, at the end of the experimental test no statistically significant differences were detected between the samples treated with the sub-thermal electric current and the controls.

Consequently, the results obtained show that the treatment with alternating electric current under sub-thermal conditions allows the build-up of fat in adipose tissue cells to be reduced and prevented.

### Example 3. Effect of treatment with alternating electric current under sub-thermal conditions on the protein expression of PPAR-γ and p-MEK in stem cells derived from adipose tissue.

PPAR-γ is a transcription factor with a crucial role in the metabolism of lipids and in adipocyte differentiation. For its part, p-MEK is a protein which interacts directly with PPAR-γ causing the inactivation and translocation thereof to the cytoplasm.

To study the action of the electric treatment on the expression and localisation of the two above-mentioned proteins, the following steps were taken:
The stem cells derived from adipose tissue obtained in accordance with example 1, in passages 3 to 7, were cultured in 60 mm Petri dishes (Nunc, Roskilde, Denmark) at a density of 2270 cells/cm² and incubated in adipogenic differentiation medium for 2 or 9 days to be treated with alternating electric current or as a culture or control group during the last 48 hours of culture, following the protocol described above.

Next, the cells present on the surface of the dish delimited by the two electrodes were collected in phosphate-buffered saline and centrifuged at 1200 rpm for 5 minutes. The sediment or pellet was lysed by treatment with a buffer containing Tris-HCI at a concentration of 10 mM, KCI at a concentration of 10 mM, dithiothreitol at a concentration of 1 mM, ethylenediaminetetraacetic acid (EDTA) at a concentration of 1 mM, phenyl methyl fluorosulphonyl (PMFS) at a concentration of 1 mM, leupeptin at a concentration of 10 µg/ml, pepstatin at a concentration of 5 µg/ml, NaF at a concentration of 100 mM, β-glycerophosphate at a concentration of 20 mM, sodium molybdate at a concentration of 20 mM, 0.5% of Triton X-100 and 0.1% of SDS for 45 minutes at 4°C. The lysates were centrifuged at 12,000 x g for 15 minutes at 4°C and the protein concentration in the supernatant was determined using the Bradford colorimetric method for quantifying proteins (Bradford MM., Anal Biochem, 1976, 72, 248-254).

The proteins obtained and quantified were separated by electrophoresis in sodium dodecyl sulphate polyacrylamide gel (SDS-PAGE), the gel being loaded with 30 µg of protein per tract and transferred to Odyssey® nitrocellulose membranes (LI-COR Biosciences, Nebraska, USA) using the semi-dry transfer methodology (Bio-Rad, Hercules, CA, USA). The membranes were blocked using phosphate-buffered saline with 5% fat-free milk powder and incubated overnight at 4°C with the rabbit monoclonal antibody to PPAR-γ 81B8 (dilution 1:1000; Cell Signalling Technology, Danvers, Massachusetts, USA), the rabbit mAb monoclonal antibody to p-MEK1/2 (dilution 1:1000; Cell Signalling Technology) or the mouse monoclonal antibody to β-actin (dilution 1:5000; Sigma-Aldrich) as a load control. The dilutions of the above-mentioned antibodies were carried out in blocking buffer (0.1% Tween and 5% fat-free milk powder in phosphate-buffered saline).

The above-mentioned antibody against PPAR-γ allowed the two isoforms of PPAR-γ of interest for the study to be detected: PPAR-γ-1 and PPAR-γ-2.

After incubation with the corresponding antibody, the membranes were washed four times with phosphate-buffered saline-Tween and then incubated for an hour at room temperature with IRDye 800CW conjugated goat anti-rabbit IgG polyclonal antibody (dilution 1:10000; LI-COR Biosciences, Nebraska, USA) or with IRDye 680LT goat anti-mouse IgG polyclonal antibody (dilution 1:15000; LI-COR Biosciences, Nebraska, USA), as appropriate. The fluorescent intensity of the strips was measured with a LI-COR Odyssey scanner (LI-COR Biosciences, Nebraska, USA) and evaluated using the Bio-Rad Quantity One computer application, version 4.6.7 (Bio-Rad, Hercules, CA, USA).

As can be seen in Fig. 2, the treatment with alternating electric current does not show an effect in the expression of PPAR-γ in cell cultures incubated for two days in adipogenic differentiation medium. However, a reduction was observed in the quantity of PPAR-γ in the cell cultures incubated for nine days in adipogenic differentiation medium and treated with alternating electric current in accordance with the protocol explained above.

In addition, Fig. 3 shows the results obtained for p-MEK which are consistent with those obtained for PPAR-γ. In this case the adipogenic differentiation process induces a reduction in the expression of the p-MEK protein. The treatment with alternating electric current does not significantly modify the expression of p-MEK in the cell cultures incubated for two days in adipogenic differentiation medium, whereas a significant increase in the expression thereof is induced in the cultures incubated for nine days.

**Example 4.** Effect of the treatment with alternating electric current under sub-thermal conditions on the cellular localisation of PPAR-γ in stem cells derived from adipose tissue.

The stem cells derived from adipose tissue obtained in accordance with example 1, in the passages 3 to 5, were cultured at the standard density on slide covers and kept under adipogenic differentiation conditions for 9 days, being subjected to the alternating electric current or control treatment, both as indicated above, for the final 48 hours.

The cells were fixed with 4% paraformaldehyde for 20 minutes at 4°C, and permeabilised with ethanol/acetic acid (95/5) at -20°C for 20 minutes. Next, the cells were incubated overnight at 4°C with a monoclonal antibody against PPAR-γ (dilution 1:50; Santa Cruz Biotechnology, TX, USA) and were marked for fluorescence with a mouse anti-IgG antibody combined with Alexa Fluor 488 (dilution 1:500; Molecular Probes, Life Technologies, MA, USA) for one hour at room temperature. The cell nuclei were counter-stained with bisBenzimide H 33258.

The results obtained are summarised in Fig. 4. A translocation of PPAR-γ was observed from the nucleus to the cytoplasm (see, in Fig. 4, the reduction in immunofluorescence of nuclear PPAR-γ in the cultures treated with alternating electric current under sub-thermal conditions). Said translocation of the protein from the nucleus to the cytoplasm shows an inactivation thereof and is consistent with all the results shown previously which demonstrate that the treatment with alternating electric current under sub-thermal conditions allows the build-up of fat in adipose tissue cells to be reduced and prevented.

**Example 5.** Effect of the treatment with alternating electric current under sub-thermal conditions on the regulation of the expression of various genes which participate in the adipocyte differentiation of stem cells derived from adipose tissue.

The stem cells derived from adipose tissue obtained in accordance with example 1, in passages 3 to 7, were cultured in 60 mm Petri dishes (Nunc, Roskilde, Denmark) at a density of 2270 cells/cm² and incubated in adipogenic differentiation medium for 2 or 9 days in order to be subjected to alternating electric current or to a treatment simulacrum (control group) during the final 48 hours of culture, following the protocol described above.

The total ribonucleic acid (RNA) of said cells was extracted using the reagent TriReagent (Sigma-Aldrich, St Louis, MO, USA) following the recommendations and protocols of the manufacturer. The cells were homogenised in 1 ml of the reagent TriReagent which contains 1 µl of glycogen (20 mg/ml, Sigma-Aldrich, St Louis, MO, USA) as a carrier for the precipitation of nucleic acids.

500 ng of total RNA were used to generate complementary deoxyribonucleic acid (DNAc) by reverse transcription using the Primer Script RT™ Reagent Kit (TaKara®, Shiga, Japan). Amplification using the polymerase chain reaction in real time was carried out using the SYBR Green I Master Kit and the LightCycler 480 II device (Roche Applied Science, Switzerland). The initial denaturation step was at 95°C for 5 minutes, followed by 45 amplification cycles at 95°C for 10 seconds, at 60°C for 15 seconds, and at 72°C for 15 seconds. The fusion curves obtained were evaluated, the products of the reaction were separated in a 2% agarose gel and finally were stained with ethidium bromide to confirm the presence of a single product. All the analyses were carried out in triplicate, and the relative quantities of the target genes were normalised against the expression of the 'housekeeping' gene RPLPO (which codifies the large ribosomal protein P0) in accordance with the ΔCt method. The primers used in the polymerase chain reactions in real time are shown in table 1.

| Gene | Primer name / Sequence number | Primer sequence (5' -> 3') |
|---|---|---|
| RPLP0 | RPLPO Fw / SEQ ID NO 1 | CCTCATATCCGGGGGAATGTG |
| | RPLP0 Rev /SEQ ID NO 2 | GCAGCAGCTGGCACCTTATTG |
| PPARG1 | PPARG1 Fw 1,1 / SEQ ID NO 3 | AAGGCCATTTTCTCAAAGA |
| | PPARG1 Rev 1,1 / SEQ ID NO 4 | AGGAGTGGGAGTGGTCTTCC |
| PPARG2 | PPARG2 Fw 1,2 / SEQ ID NO 5 | CCATGCTGTTATGGGTGAAA |
| | PPARG1 Rev 1,2 / SEQ ID NO 6 | TCAAAGGAGTGGGAGTGGTC |
| FABP4 | FABP4 Fw 1.2 / SEQ ID NO 7 | AGCACCATAACCTTAGATGGGG |
| | FABP4 Rev 1.2 / SEQ ID NO 8 | CGTGGAAGTGACGCCTTTCA |
| SCD | SCD Fw 1.1 / SEQ ID NO 9 | TCTAGCTCCTATACCACCACCA |
| | SCD Rev 1.1 / SEQ ID NO 10 | TGTCGTCTTCCAAGTAGAGGG |
| PLIN | Plin Fw / SEQ ID NO 11 | GTGGAGTACCTCCTCCCTG |
| | Plin Rev /SEQ ID NO 12 | GGTGTATCGAGAGAGGGTGTT |
| ANGPTL4 | ANGPTL4 Fw 1.2 / SEQ ID NO 13 | GGCTCAGTGGACTTCAACCG |
| | ANGPTL4 Rev 1.2 / SEQ ID NO 14 | CCGTGATGCTATGCACCTTCT |
| SREBP1c | SREBP1c Fw 1.1 / SEQ ID NO 15 | ACCGACATCGAAGGTGAAGT |
| | SREBP1c Rev 1.1 / SEQ ID NO 16 | AGCATGTCTTCGAAAGTGCA |
| FASN | FASN Fw 1.1 / SEQ ID NO 17 | TACGTACTGGCCTACACCCAGA |
| | FASN Rev 1.1 / SEQ ID NO 18 | TGAACTGCTGCACGAAGAAGCATAT |

The results obtained are summarised in Fig. 5 and 6.

In Fig. 5 it can be seen that as the adipogenic process progresses the expression of the following genes increases: PPARG1, PPARG2, FABP4, PLIN, ANGPTL4, SREBP1c, SCD and FASN.

In Fig. 6 it can be seen that the treatment with alternating electric current under sub-thermal conditions does not produce a statistically significant effect on the expression of any of the genes analysed in the cell cultures incubated for two days in adipogenic differentiation medium. However, a reduction is observed in the expression of the genes PPARG1, PLIN, ANGPTL4 and FASN in the cell cultures incubated for nine days in adipogenic differentiation medium and treated with alternating electric current in accordance with the protocol explained earlier. No statistically significant variation was observed for the rest of the genes. The results obtained are consistent with all the results shown previously which demonstrate that the treatment with alternating electric current under sub-thermal conditions allows the build-up of fat in adipose tissue cells to be reduced and prevented.

Although the invention has been presented and described with reference to embodiments and examples thereof, it will be understood that said embodiments and examples do not limit the invention, and many structural or other details which will be clear to persons skilled in the art after interpreting the subject matter which is disclosed in the present description, claims and drawings may therefore vary. Thus, all variants and equivalents will be included within the scope of the present invention if said variants and equivalents may be considered to fall within the most extensive scope of the following claims.

## Claims

1. Method for reducing or preventing the build-up of adipose tissue in an area of a human body, having a solely and exclusively cosmetic effect **characterised in that** it comprises the application in said area of the human body of alternating electric current with a frequency in the range 0.4 to 0.6 MHz under sub-thermal conditions, in which the density of the alternating electric current is between 1 and 3000 µA/mm² and each of the application sessions of alternating electric current lasts between 30 and 90 minutes.

2. Method according to claim 1, **characterised in that** the human body is an adult human body.

3. Method according to either claim 1 or claim 2, **characterised in that** the alternating electric current has a frequency in the range 0.4 MHz to 0.5 MHz.

4. Method according to claim 3, **characterised in that** the alternating electric current has a frequency of 0.448 MHz.

5. Method according to any of the preceding claims, **characterised in that** the density of the alternating electric current is between 5 and 500 µA/mm².

6. Method according to any one of the preceding claims, **characterised in that** in the method between 6 and 20 application sessions of alternating electric current are carried out.

7. Method according to claim 6, **characterised in that** in the method between 8 and 15 application sessions of alternating electric current are carried out.

8. Method according to either claim 6 or claim 7, **characterised in that** between 1 and 5 application sessions of alternating electric current are applied per week.

9. Method according to claim 8, **characterised in that** three application sessions of alternating electric current are applied per week.

10. Method according to any one of the preceding claims, **characterised in that** each of the application sessions of alternating electric current lasts for 60 minutes.

11. Method according to any one of the preceding claims, **characterised in that** the area of the human body is the abdomen, gluteus, thighs, waist, back, arms, 'saddlebags' or combinations thereof.
